# EUROPÄISCHE PATENTSCHRIFT

(11) **EP 1 392 184 B1**
(45) Veröffentlichungstag und Bekanntmachung des Hinweises auf die Patenterteilung: **03.09.2008**
(21) Anmeldenummer: 02716729.5
(22) Anmeldetag: 06.02.2002
(51) Int. Cl.: A61B 17/32

(54) **UROLOGISCHES RESEKTOSKOP FÜR KINDER**
UROLOGICAL RESECTOSCOPE FOR CHILDREN
RESECTOSCOPE UROLOGIQUE POUR ENFANTS

(30) Priorität: 30.05.2001 DE 10126541
(43) Veröffentlichungstag der Anmeldung: 03.03.2004
(73) Patentinhaber: OLYMPUS WINTER & IBE GMBH, 22045 Hamburg (DE)
(72) Erfinder: WOSNITZA, Thomas, 21337 Lüneburg (DE); DICKOPP, Jörg, 21465 Reinbeck (DE)
(74) Vertreter: Schaefer, Konrad
(86) Internationale Anmeldenummer: PCT/EP2002/001198
(87) Internationale Veröffentlichungsnummer: WO 2002/096305

(56) Entgegenhaltungen:
- US-A- 4 648 399
- US-A- 5 088 998
- US-A- 5 287 845

## Beschreibung

Die Erfindung betrifft ein urologisches Resektoskop der im Oberbegriff des Anspruches 1 genannten Art.

Solche Resektoskope müssen zum Einsatz in der noch sehr engen Harnröhre eines Kindes mit einem besonders engen Schaftrohr ausgebildet sein. Das Schaftrohr umschließt daher mit formangepaßtem Querschnitt, der etwa birnenförmig ausgebildet ist, die Querschnitte der Betätigungsstange und des Optikrohres. Aus Querschnittsgründen wird hier sogar auf die übliche Doppelrohrausbildung bei Resektoskopen zur Schaffung eines zweiten Spülkanals verzichtet.

Ein gattungsgemäßes Resektoskop in Ausführung für Kinder ist aus dem Katalog "Transurethrale Resektion" der Anmelderin aus dem Jahr 1997, Seiten 30 - 32 bekannt. Dabei zeigt die Katalognummer A3753 auf Seite 32 einen Arbeitseinsatz, bei dem die chirurgische Arbeitseinrichtung als Schneidschlinge ausgebildet ist. In Bezug auf das die Arbeitsrichtung unten definierende Griffstück muß hierbei die Schneidschlinge oben befestigt sein, die den Elektrodenträger ausbildende Betätigungsstange also oberhalb des Optikrohres verlaufen. Sie verläuft bei der bekannten Konstruktion oberhalb des Optikrohres durch den Hauptkörper bis zum Schiebestück und ist in diesem Schiebestück, das an dem Resektoskop, das ohne das zugehörige Schaftrohr A3750 mit der Katalognummer A3752 dargestellt ist, oberhalb des Optikrohres lösbar befestigt und elektrisch kontaktiert.

Bei Operationen mit einem urologischen Resektoskop für Kinder wird neben einer HF-beaufschlagten Schneidschlinge stets auch ein kaltes Messer mit von der Betätigungsstange abstehender Schneidklinge benötigt. Aus operationstechnischen Gründen ist es hierbei jedoch erforderlich, daß die Schneidklinge von unten nach oben ragt. Daher muß die Betätigungsstange bei dieser Ausführung, die in der erwähnten Katalogseite mit der Artikelnummer A3748 gezeigt ist, unterhalb des Optikrohres angeordnet sein. Es ist nach dem Stand der Technik ein zweites Resektoskop erforderlich, das ohne das zugehörige Schaftrohr A3744 mit der Katalognummer A3745 dargestellt ist und bei dem die Betätigungsstange unterhalb des Optikrohres bis zum Schiebekörper läuft und an diesem befestigt ist.

Nachteilig dabei sind die erheblichen Mehrkosten, da das komplette Resektoskop in zwei Ausführungen bereitgestellt werden muß.

Die Aufgabe der vorliegenden Erfindung besteht darin, ein gattungsgemäßes Resektoskop zu schaffen, bei dem die Kosten bei Verwendung beider chirurgischer Arbeitsmethoden geringer sind.

Diese Aufgabe wird mit den Merkmalen des Anspruches 1 gelöst.

Erfindungsgemäß wird die Betätgigungsstange in einem Freiraum des Hauptkörpers aus ihrer Winkellage am Schiebekörper in die Winkellage im Schaftbereich umgelenkt. Nur hier, nämlich im Hauptkörper, besteht aus konstruktiven Gründen dazu Gelegenheit, da eine Umlenkung proximal vom Hauptkörper den Schiebebereich des Schiebekörpers verkürzen würde und für eine Umlenkung im engen Schaftrohr kein Platz ist. Es wird ein Resektoskop geschaffen, in dem nur das Schaftrohr gewechselt bzw. in anderer Winkellage montiert werden muß. Ist die erste Winkellage, also die Befestigung der Betätigungsstange am Schiebekörper, z.B. oberhalb des Optikrohres gelegen, so kann die Betätigungsstange für das kalte Messer mit Umlenkstelle ausgebildet sein, wogegen die Betätigungsstange für die HF-Schneidschlinge ohne Umlenkstelle konventionell und gerade ausgebildet ist. Der größte Teil des sehr teuren Resektoskopes kann somit für beide chirurgische Arbeitsmethoden verwendet werden. Lediglich die Betätigungsstange mit der chirurgischen Arbeitseinrichtung (HF-Schneidschlinge oder kaltes Messer) müssen gewechselt werden, was ohnehin erforderlich ist. Ebenso muß der Schaft gewechselt bzw. gedreht werden.

Vorteilhaft sind die Merkmale des Anspruches 2 vorgesehen. Das Führungsrohr sichert im Bereich des Freiraumes im Hauptkörper eine sichere Führung der Betätigungsstange am Optikrohr, so daß störende seitliche Ausweichbewegungen vermieden werden. Dieses Führungsrohr kann z.B. zusätzlich zu einem weiteren Führungsrohr vorgesehen werden, das bei Geräten nach dem Stand der Technik die Betätigungsstange im Schaftbereich auf dem Optikrohr führt.

Dabei sind vorteilhaft die Merkmale des Anspruches 3 vorgesehen. Diese Konstruktion der Umlenkstelle ist besonders raumsparend, da außer dem sehr dünnwandigen Führungsrohr an der Umlenkstelle nur in den beiden Winkellagen Teile der Betätigungsstange vorgesehen sind. Ein zusätzlich raumfordemder, um das Optikrohr verschwenkter Zwischenbereich der Betätigungsstange entfällt.

In der Zeichnung ist die Erfindung beispielsweise und schematisch dargestellt. Es zeigen:
- Figur 1: eine teilgeschnittene Seitenansicht eines erfindungsgemäßen Resektoskopes mit HF-Schneidschlinge,
- Figur 2: einen Schnitt nach Linie 2-2 in Figur 1,
- Figur 3: eine Teildarstellung der Figur 1 in Ausführung mit kaltem Messer,
- Figur 4: einen Schnitt nach Linie 4-4 in Figur 3,
- Figur 5: eine Darstellung der Umlenkstelle der Betätigungsstange gemäß Figur 3 in einer anderen Ausführungsform,
- Figur 6: einen Schnitt nach Linie 6-6 in Figur 1 und
- Figuren 7 und 8: Darstellungen gemäß Figur 6 in anderen Ausführungsformen des Resektoskopes.

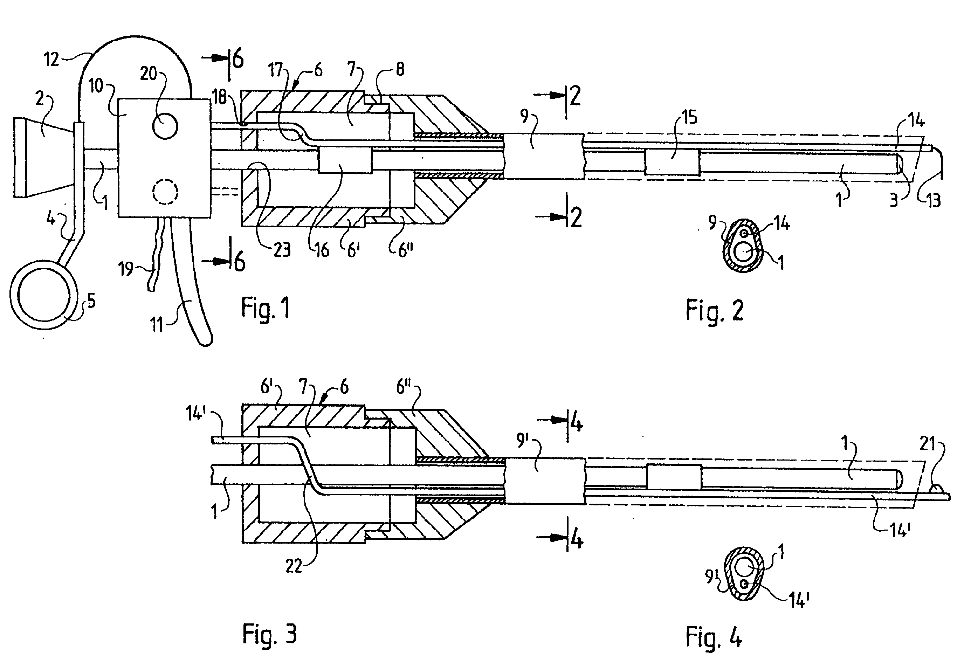

Figur 1 zeigt in teilgeschnittener Seitenansicht ein erfindungsgemäßes Resektoskop. Ein Optikrohr 1 nimmt, ggf. zu Reinigungszwecken herausziehbar, eine Optik mit proximalem Okular 2 und distalem Objektiv 3 auf. Am proximalen Ende des Optikrohres 1 ist eine Optikführungsplatte 4 befestigt, die unten einen Daumenring 5 trägt.

Im Abstand zur Optikführungsplatte 4 ist auf dem Optikrohr 1 ein Hauptkörper 6 befestigt, der um das Optikrohr 1 herum einen Freiraum 7 ausbildet. Im Ausführungsbeispiel ist dazu der Hauptkörper 6 als Hohlgehäuse ausgebildet.

Auf einem Flansch 8 am distalen Ende des Hauptkörpers 6 ist ein in größeren Teilen seiner Erstreckung gestrichelt dargestelltes Schaftrohr 9 lösbar befestigt.

In dem in den Figuren 1 und 3 dargestellten Ausführungsbeispiel besteht der Hauptkörper 6 aus zwei Teilen 6', 6", die an dem Flansch 8 miteinander verbindbar sind. Der proximal gelegenen Hauptkörper 6' ist auf dem Optikrohr 1 befestigt. Der abnehmbare distale gelegene Teile 6" ist am Schaftrohr 9 befestigt und bildet dessen Kupplungsteil zur Verbindung mit dem Hauptkörper 6.

Ein Schiebekörper 10 trägt auf seiner Unterseite ein Griffstück 11 für den Zeigefinger des Operateurs und ist zwischen Optikführungsplatte 4 und Hauptkörper 6 verschiebbar auf dem Optikrohr 1 gelagert. Eine Blattfeder 12 verbindet die Optikführungsplatte 4 und den Schiebekörper 10.

Die Griffstellung der Hand des Operateurs unterhalb des dargestellten Resektoskopes definiert an diesem die Richtung "unten". Durch Anfassen mit Daumen und Zeigefinger am Daumenring 5 und dem Griffstück 11 kann der Schiebekörper 10 gegen die Rückstellkraft der Blattfeder 12 bewegt werden.

In Figur 1 ist das dargestellte Resektoskop zum Hochfrequenzschneiden mit einer Schneidschlinge 13 ausgerüstet, die an einer den Elektrodenträger bildenden Betätigungsstange 14 sitzt. Aus schneidtechnischen Gründen muß die Schneidschlinge 13 von oben vor den Sichtbereich des Objektives 3 ragen. Die Betätigungsstange 14 muß daher im Bereich des Schaftrohres 9 oberhalb des Optikrohres 1 verlaufen. Sie ist auf diesem mit einem Führungsrohr 15 längsverschiebbar geführt.

Wie Figur 2 im Querschnitt zeigt, umschließt das Schaftrohr 9 mit engem birnenförmigem Querschnitt die eng aneinanderliegenden Querschnitte der Betätigungsstange 14 und des Optikrohres 1. Damit wird der geringstmögliche Umfang für das Schaftrohr 9 sichergestellt.

Wie Figur 1 zeigt, durchläuft die Betätigungsstange 14 in proximaler Richtung parallel und eng dem Optikrohr 1 anliegend den Bereich des Schaftrohres 9 bis in den Freiraum 7 des Hauptkörpers 6 hinein. Dort ist sie mit einem weiteren Führungsrohr 16 zusätzlich sichernd auf dem Optikrohr 1 geführt und vergrößert dort mit einem Verschwenkungsstück 17 ihren Abstand zum Optikrohr 1, um dann parallel zu diesem durch eine Bohrung 18 in der proximalen Stirnwand des Hauptkörpers 6 zum Schiebekörper 10 zu verlaufen. Dort ist das proximale Endstück der Betätigungsstange 14 in eine Aufnahmebohrung eingesteckt, auf nicht dargestellte Weise mit einem elektrischen Anschlußkabel 19 kontaktiert und z.B. mit der dargestellten Klemmschraube 20 befestigt.

Figur 3 zeigt in distaler Teilansicht das Resektoskop der Figur 1, das also einschließlich des Hauptkörpers 6 und in seinen proximal von diesem gelegenen Teilen mit der Ausführungsform der Figur 1 identisch ist.

Das Resektoskop ist in der Ausführung der Figur 3 zum kalten Schneiden mit einer Schneidklinge 21 ausgebildet, die aus chirurgischen Gründen von unterhalb des Optikrohres 1 nach oben ragen muß, wie dies in Figur 3 dargestellt ist. Daher muß in dem ebenfalls sehr engen Schaftrohr 9', dessen Querschnitt in Figur 4 dargestellt ist, die Betätigungsstange 14' unterhalb des Optikrohres 1 das Schaftrohr 9' durchlaufen.

Da der proximale Endbereich des Resektoskopes mit dem der Figur 1 übereinstimmt und somit auch die Befestigung der Betätigungsstange 14' oberhalb des Optikrohres erfolgt, ist die Betätigungsstange 14' im Freiraum 7 des Hauptkörpers 6 an einer Umlenkstelle 22 seitlich um das Optikrohr 1 herum aus dem Abstand zum Optikrohr, den sie im Schaftrohr 9' einnimmt, und der Winkellage in diesem Bereich (unten) in den erforderlichen Abstand und die Winkellage (in größerem Abstand und oben) am Schiebekörper 10 umgelenkt. Die Umlenkstelle 22, die um das Optikrohr 1 herum Raum benötigt, liegt im Freiraum 7, der von derartiger axialer Länge ist, daß er die Verschiebung der Umlenkstelle 22 mit der Verschiebung des Schiebekörpers 10 zuläßt.

Der Hauptkörper 7 ist wie in den Figuren dargestellt, in beiden Teilen 6', 6" des Hauptkörpers 6 ausgebildet. Er kann in anderer Ausführungsform in nur einem der beiden Teile, z. B. in dem das Schaftrohr 9 tragenden Teil 6" ausgebildet sein.

Bei der Ausführung der Figur 3 muß, wie der Vergleich der Figuren 2 und 4 zeigt, das Optikrohr 9' gegenüber dem Optikrohr 9 der Ausführungsform der Figur 1 ausgewechselt werden. Es könnte auch dasselbe Rohr in um 180° verdrehter Winkellage verwendet werden.

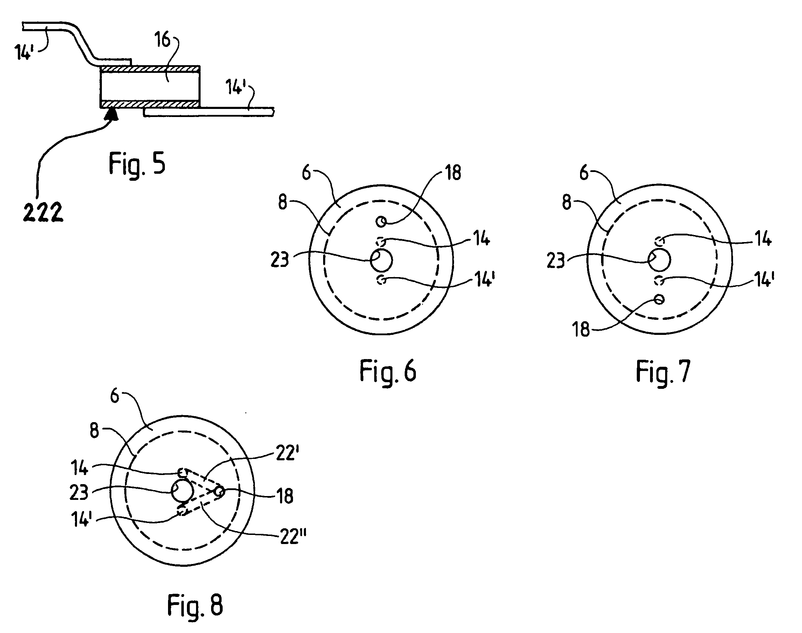

Figur 5 zeigt eine Umlenkstelle 222 in anderer Ausführungsform. Es ist hier das bereits in Figur 1 gezeigte Führungsrohr 16 vorgesehen, an dem die Betätigungsstange 14' der Figur 2 in geteilter Ausführung befestigt ist. Wie Figur 5 zeigt, ist ein proximaler Teil der Betätigungsstange 14' von oben am Führungsrohr 16 befestigt, z.B. verlötet, und ein distales Stück von unten. Dadurch wird das in Figur 3 dargestellte seitliche Umlaufen des Optikrohres 1 an der Umlenkstelle 222 und der damit erforderliche seitliche Platzbedarf eingespart.

Figur 6 zeigt im Schnitt nach Linie 6-6 in Figur 1 die Ansicht der proximalen Endfläche des Hauptkörpers 6 mit einer mittleren Bohrung 23, in der das Optikrohr durchgesteckt und befestigt ist. Ferner ist die Bohrung 18 dargestellt, durch die die Betätgigungsstange 14 bzw. 14' proximal zum Schiebekörper 10 verläuft. Gestrichelt ist auch der distal gelegene Flansch 8 des Hauptkörpers 6 dargestellt. Ferner sind gestrichelt die Positionen der Betätigungsstange 14 gemäß Figur 1 und 14' gemäß Figur 3 dargestellt.

In Figur 7 wird eine Ausführungsvariante des Resektoskopes erläutert, bei der im Unterschied zur vorerwähnten Ausführungsform die Befestigung der Betätigungsstange am Schiebekörper 10 unterhalb des Optikrohres 1 liegt, wie dies in Figur 1 mit gestrichelter Darstellung der Betätigungsstange und gestrichelter Darstellung der Klemmschraube 20 angedeutet ist. Die Bohrung 18 liegt bei dieser Ausführungsform, wie Figur 7 zeigt, unterhalb der Bohrung 23. Würde hier die Betätigungsstange 14 mit distaler Schneidschlinge verwendet, die proximal des Hauptkörpers 6, die in Figur 7 dargestellt Lage haben muß, so muß diese innerhalb des Hauptkörpers 6 mit der z.B. in Figur 3 gezeigten Umlenkung 22 versehen sein, um von dort nach unten umgelenkt durch die Bohrung 18 zu unten liegenden Befestigung am Schiebekörper 10 verlaufen zu können. Wird die Betätigungsstange 14' mit Messerklinge 21 verwendet, so könnte diese innerhalb des Hauptkörpers 6 mit dem einfachen Verwenkungsstück 17 verschwenkt durch die Bohrung 18 verlaufen.

Figur 8 zeigt eine weitere Ausführungsform des Resektoskopes in Darstellung gemäß den Figuren 6 und 7. Bei dieser Ausführungsform des Resektoskopes liegt in dem nicht dargestellten Schiebekörper 10 die Befestigung für die Betätigungsstange seitlich vom Optikrohr 1, also beispielsweise gemäß Figur 1 gegenüber der Zeichnungsebene nach vom zum Betrachter hin versetzt. Folglich ist hier die zum Schiebestück 10 führende Bohrung 18 seitlich angeordnet, also neben der das Optikrohr aufnehmenden Bohrung 23. Im Schaftbereich sind hier wiederum die oben liegende Betätigungsstange 14 oder die unten liegende Betätigungsstange 14' erforderlich, je nach dem, ob die HF-Schneidmethode gemäß Figur 1 oder die kalte Schneidmethode gemäß Figur 3 verwendet wird. Im Hauptkörper 6, also in dessen Freiraum 7 sind sowohl für die Betätigungsstange 14 als auch für die Betätigungsstange 14' Umlenkstellen 22' bzw. 22" vorzusehen, die die Abstandsversetzung zum Optikrohr 1 und die Winkelverschwenkung von 90° zur einen oder 90° zur anderen Seite bewirken.

Die dargestellte Konstruktion stellt ein Resektoskop mit sogenanntem aktivem Transporteur dar, bei dem also gegen die Kraft der Blattfeder 12 die Schneidschlinge 13 von Hand zurückgezogen wird, während sie mit der Kraft der Blattfeder 12 von allein wieder nach vom geschoben wird. Vielfach werden auch sogenannte passive Transporteure bevorzugt. Das erfindungsgemäße Resektoskop kann auch in dieser Weise ausgebildet sein. Dazu wäre der Daumenring 5 am Schiebekörper 10 und das Griffstück 11 am Hauptkörper 6 zu befestigen. Die Blattfeder 12 wäre zwischen dem Schiebekörper 10 und dem Hauptkörper 6 vorzusehen. Dann wird die Schneidschlinge 13 gegen die Kraft der Feder vorgeschoben und von dieser selbsttätig zurückgedrückt (passives Schneiden).

Im dargestellten Ausführungsbeispiel und der soeben erläuterten Variante ist die Blattfeder 12 als die beiden von ihr verbundenen Teile auseinanderdrückende Feder ausgebildet. Sie kann jedoch auch als zusammenziehende Feder ausgebildet sein. Dann wäre sie im dargestellten Ausführungsbeispiel gemäß Figur 1 bei unveränderter Anordnung von Daumenring 5 und Griffstück 11 zwischen dem Schiebekörper 10 und dem Hauptkörper 6 vorzusehen. Diese Ausführung kann auch bei dem vorerwähnten passiven Transporteur vorgesehen sein.

## Patentansprüche

1. Urologisches Resektoskop für Kinder, mit einem Hauptkörper (6), an dem ein sich nach distal erstreckendes Schaftrohr (9, 9') ankoppelbar ist, und mit einer in festem Abstand proximal zum Hauptkörper angeordneten Optikführungsplatte (4), wobei zwischen Hauptkörper und Optikführungsplatte ein ein Griffstück (11) tragender Schiebekörper (10) verschiebbar geführt ist und wobei am Schiebekörper eine Betätigungsstange (14, 14') in einem ersten Abstand zu einem das Resektoskop längs durchlaufenden Optikrohr (1) und in einer ersten Winkellage zum Griffstück (11) lösbar befestigt ist, die vom Schiebekörper aus parallel zum Optikrohr, durch den Hauptkörper und sodann in einer zweiten Winkellage und in einem zweiten sehr geringen Abstand zum Optikrohr parallel zu diesem durch das die Querschnitte der Betätigungsstange und des Optikrohres mit angepaßtem Querschnitt eng umschließende Schaftrohr bis in dessen distalen Endbereich läuft, wo die Betätigungsstange eine chirurgische Arbeitseinrichtung (13, 21) trägt, **dadurch gekennzeichnet, daß** im Hauptkörper (6) ein Freiraum (7) einer wenigstens dem Verschiebeweg der Betätigungsstange (14, 14') entsprechenden Länge ausgebildet ist, wobei die Betätigungsstange im Bereich des Freiraumes eine Umlenkstelle (22, 222, 22', 22") aufweist, an der sie um das Optikrohr (1) herum aus der ersten Winkellage und dem ersten Abstand zu diesem in die zweite Winkellage und den zweiten Abstand verschwenkt ist.

2. Resektoskop nach Anspruch 1, **dadurch gekennzeichnet, daß** an der Umlenkstelle (222) ein auf dem Optikrohr (1) laufendes Führungsrohr (16) an der Betätigungsstange (14, 14') befestigt ist.

3. Resektoskop nach Anspruch 2, **dadurch gekennzeichnet, daß** die Betätigungsstange (14') an der Umlenkstelle (222) geteilt ist, wobei an dem Führungsrohr (16) der proximale Teil der Betätigungsstange in der ersten Winkellage und der distale Teil in der zweiten Winkellage befestigt ist.

## Claims

1. A neurological resectoscope for children with a main body (6), to which a distally extending tubular shaft (9,9') may be coupled, and with an optical system guide plate (4), which is disposed at a fixed spacing proximally of the main body, wherein a sliding body (10) carrying a handle (11) is slidably guided between the main body and the optical system guide plate and wherein releasably secured to the sliding body at a first spacing from an optical system tube (1) extending longitudinally through the resectoscope and in a first angular position with a respect to the handle (11) there is an actuating rod (14,14'), which extends from the sliding body parallel to the optical system tube, through the main body and then in a second angular position and at a second very small spacing from the optical system tube and parallel to it through the tubular shaft, which closely surrounds the cross sections of the actuating rod and of the optical system tube with a matched cross section to the distal end region of the tubular shaft, where the actuating rod carries a surgical working device (13,21), **characterised in that** formed in the main body (6) there is a space (7) of a length corresponding to at least the displacement travel of the actuating rod (14,14'), wherein the actuating rod has, in the region of the space, a deflection point (22,222,22',22"), at which it is bent around the optical system tube (1) out of the first angular position and the first spacing from it into the second angular position and the second spacing.

2. A resectoscope as claimed in claim 1, **characterised in that** at the deflection point (222), a guide tube (16) extending on the optical system tube (1) is fastened to the actuating rod (14,14-).

3. A resectoscope as claimed in claim 2, **characterised in that** the actuating rod (14') is divided at the deflection point (222), wherein the proximal portion of the actuating rod is fastened to the guide tube (16) in the first angular position and the distal portion is fastened to it in the second angular position.

## Revendications

1. Résectoscope urologique pour enfants, avec un corps principal (6) auquel peut être accouplé une gaine (9, 9') s'étendant dans le sens distal, et avec une plaque de guidage de l'optique (4) agencée du côté proximal du corps principal à une distance fixe de celui-ci, un corps coulissant (10) portant une poignée (11) étant guidé de façon à pouvoir se déplacer entre le corps principal et la plaque de guidage de l'optique, et une barre de commande (14, 14') étant fixée de façon amovible au corps coulissant à une première distance d'un tube optique (1) traversant longitudinalement le résectoscope et dans une première position angulaire par rapport à la poignée (11), cette barre de commande sortant du corps coulissant parallèlement au tube optique pour traverser le corps principal puis, en une seconde position angulaire et à une seconde distance très faible du tube optique et parallèlement à celui-ci, la gaine, dont la section appropriée enveloppe étroitement les sections de la barre de commande et du tube optique, jusqu'à la partie d'extrémité distale de la gaine où la barre de commande porte un dispositif d'opération chirurgical (13, 21), **caractérisé en ce qu'**est réservé dans le corps principal (6) un espace libre (7) d'une longueur correspondant au moins à celle sur laquelle se déplace la barre de commande (14, 14'), la barre de commande présentant au niveau de l'espace libre une déviation (22, 222, 22', 22") au niveau de laquelle elle passe de l'autre côté du tube optique (1) et de la première position angulaire et la première distance à la seconde position angulaire et la seconde distance par rapport au tube optique.

2. Résectoscope selon la revendication 1, **caractérisé en ce qu'**un tube guide (16) coulissant sur le tube optique (1) est fixé à la barre de commande (14, 14') au niveau de la déviation (222).

3. Résectoscope selon la revendication 2, **caractérisé en ce que** la barre de commande (14') est divisée en deux parties au niveau de la déviation (222), la partie proximale de la barre de commande étant fixée au tube guide (16) dans la première position angulaire et la partie distale y étant fixée dans la seconde position angulaire.
